# EUROPEAN PATENT SPECIFICATION

(11) **EP 2 683 042 B1**
(45) Date of publication and mention of the grant of the patent: **23.09.2020**
(21) Application number: 13174820.4
(22) Date of filing: 03.07.2013
(51) Int. Cl.: H01T 23/00

(54) **Ionizer**
Ionisator
Ioniseur

(30) Priority: 05.07.2012 KR 20120073441
(43) Date of publication of application: 08.01.2014
(73) Proprietor: LG Electronics Inc., Seoul 150-721 (KR)
(72) Inventor: Lee, Yanghwa, Seoul 153-023 (KR); Park, Hyungho, Seoul 153-023 (KR); Lee, Sunghwa, Seoul 153-023 (KR); Shin, Jinhyouk, Seoul 153-023 (KR)
(74) Representative: Vossius & Partner Patentanwälte Rechtsanwälte mbB

(56) References cited:
- EP-A1- 2 187 139
- JP-A- 2002 102 683
- US-A1- 2010 175 391

## Description

The present invention relates to an ionizer, and more particularly to an efficient and safe ionizer.

An air conditioner is an apparatus that generally cools or heats an indoor space using a refrigeration cycle constituted by a compressor, an outdoor heat exchanger, an expansion valve, and an indoor heat exchanger. That is, the air conditioner may be configured with a cooler to cool the indoor space, and a heater to heat the indoor space. In addition, the air conditioner may be an air conditioner that is capable of both heating and cooling the indoor space.

The indoor unit of such an air conditioner is provided with a dust collector to collect and eliminate foreign substances such as dust floating in the air. The dust collector may come in various shapes. Recently, an electric dust collector that collects foreign substances by charging the foreign substances is used.

US 2010/0175391 A1 describes an ionizer according to the first part of the independent claims. Further related art is shown in JP 2002 102683 A or EP 2 187 139 A1.

Therefore, the present invention has been made in view of the above problems, and it is an object of the present invention to provide an efficient ionizer.

It is another object of the present invention to provide a safe ionizer.
These objects are achieved with an ionizer according to the claims.

The above and other objects, features and other advantages of the present invention will be more clearly understood from the following detailed description taken in conjunction with the accompanying drawings, in which:
FIG. 1 is a front view showing an air conditioner according to an exemplary embodiment of the present invention;
FIG. 2 is a cross-sectional view showing the air conditioner of FIG. 1.
FIG. 3 is a front view showing a dust collector according to one embodiment of the present invention;
FIG. 4 is a partial cross-sectional view showing the dust collector shown in FIG. 3;
FIG. 5 is a perspective view showing an ionizer according to an embodiment of the present invention;
FIG. 6 is a detailed view showing a part of the ionizer shown in FIG. 5;
FIG. 7 is a front view showing a dust collector according to another embodiment of the present invention;
FIG. 8 is a perspective view showing an air conditioner according to another embodiment of the present invention;
FIG. 9 is a cross-sectional view showing the air conditioner shown in FIG. 8; and
FIG. 10 is a perspective view showing a dust collector according to anther embodiment of the present invention.

Reference will now be made in detail to the preferred embodiments of the present invention, examples of which are illustrated in the accompanying drawings. Wherever possible, the same reference numbers will be used throughout the drawings to refer to the same or like parts.

Hereinafter, a description will be given of an ionizer of the present invention with reference to the drawings.

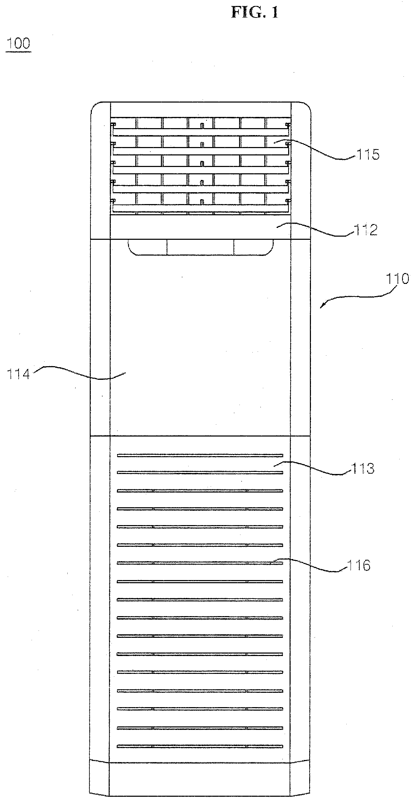

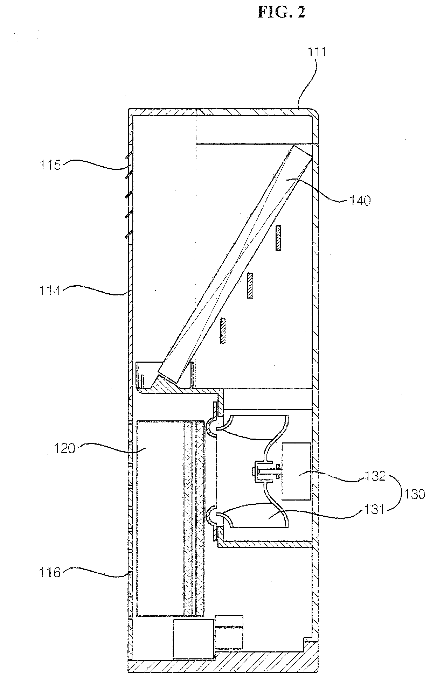

FIG. 1 is a front view showing an air conditioner according to an exemplary embodiment of the present invention, and FIG. 2 is a cross-sectional view showing the air conditioner of FIG. 1.

The air conditioner 100 includes a cabinet 110, a dust collector 120 disposed inside the cabinet 110 to remove foreign substances from the moving air, an air blower 130 to force the air to flow, and a heat exchanger 140 to cause heat to be exchanged between the air blown by the air blower 130 and a refrigerant to adjust temperature. In the illustrated embodiment, the air conditioner 100 is a floor standing indoor unit.

The cabinet 110 includes a cabinet body 111, a cabinet cover 114 disposed at and coupled to the front of the cabinet body 111, a lower front panel 113 coupled to the cabinet body 111 and provided with an air suction portion 116 allowing external air to be suctioned into the cabinet 110 therethrough, and an upper front panel 112 coupled to the cabinet body 111 and provided with an air discharge portion 115 allowing the air in the cabinet 110 to be discharged to the outside.

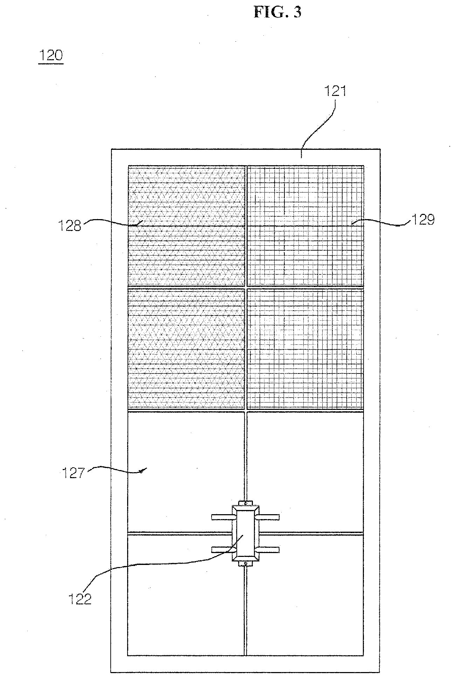

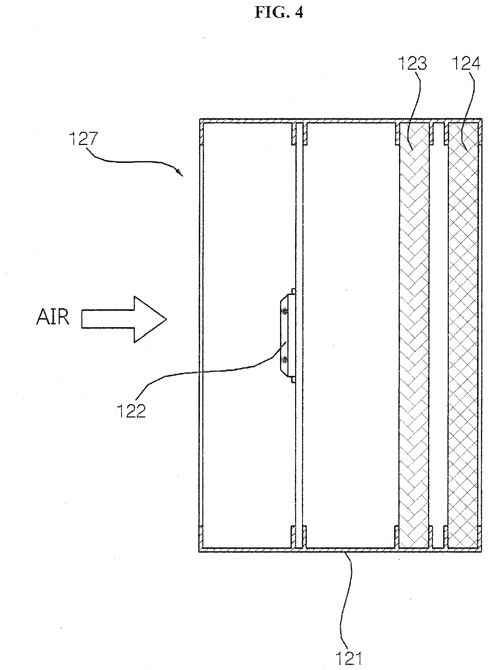

The dust collector 120 ionizes molecules in the air, charges foreign substances with produced ions, and collects the charged foreign substances. The dust collector 120 will be described later in detail with reference to FIGS. 3 and 4 .

The air blower 130 is disposed in the cabinet 110. The air blower 130 blows air such that air outside the cabinet 110 is suctioned into the cabinet 110, passes through the dust collector 120 and the heat exchanger 140, and is then discharged outside the cabinet 110. The air blower 130 includes a motor 132 to generate rotational power, and a fan 131 rotated by the motor 132.

The heat exchanger 140 causes heat exchange to occur between the air and a refrigerant to cool or heat the air. When the refrigerant evaporates in the heat exchanger 140, the air is cooled. When the refrigerant condenses, the air is heated. The heat exchanger 140 may include a pipe (not shown) through which the refrigerant flows, and a cooling fin (not shown) coupled to the pipe. The heat exchanger 140 is formed of a metallic material.

The air flows as follows. When the fan 131 is rotated by operation of the motor 132, the external air is introduced into the cabinet 110 through the air suction portion 116. While air introduced into the cabinet 110 passes through the dust collector 120, foreign substances are removed therefrom. The air from which the foreign substances are removed is transferred to the heat exchanger 140 according to rotation of the fan 131. The air is cooled or heated when it exchanges heat with the refrigerant in the heat exchanger 140. The air having exchanged heat with the refrigerant is discharged from the cabinet 110 through the air discharge portion 115.

FIG. 3 is a front view showing a dust collector according to one embodiment of the present invention, and FIG. 4 is a partial cross-sectional view showing the dust collector shown in FIG. 3.

The dust collector 120 according to the illustrated embodiment includes a dust collector case 121 forming a flow channel allowing air to flow therethrough, an ionizer 122 to ionize molecules in the air to produce ions, a charged dust collecting filter 123 to collect foreign substances charged by ions produced by the ionizer 122, and a grounded ion trap 124 to collect the ions.

A flow channel is formed in the dust collector case 121 to allow the air suctioned through the air suction portion 116 to flow to the air blower 130. The dust collector case 121 is formed in a hollow shape to allow the air suction portion 116 to communicate with the air blower 130. In the illustrated embodiment, the dust collector case 121 is formed in a hexahedral shape having open opposite faces.

In the illustrated embodiment, the dust collector case 121 is partitioned into a plurality of sections, and the ionizer 122 is disposed in one of the sections. The ionizer 122 is preferably disposed at the center of a dust collecting section 127 at the lower portion of the dust collector case 121.

A sterilization filter 128 to eliminate germs and/or a deodorization filter 129 to eliminate odor may be disposed at a portion where the ionizer 122 is not disposed. In the illustrated embodiment, the sterilization filer 128 and the deodorization filter 129 are disposed at an upper portion of the dust collector case 121.

That is, the dust collector case 121 is partitioned into sections, by a cross sectional plane perpendicular to the air flow direction, such that different functions are performed in each section . One of the sections is a dust collecting section 127, in which the ionizer 122, the dust collecting filter 123, and the ion trap 124 may be disposed. In another section, the sterilization filter 128 may be disposed. In a further section, the deodorization filter 129 may be disposed.

In the illustrated embodiment, the ionizer 122, the sterilization filter 128, and the deodorization filter 129 are disposed in the same plane perpendicular to the air flow direction. However, in another embodiment, they may be sequentially disposed along the air flow direction. That is, the sterilization filter 128 and the deodorization filter 129 are sequentially disposed in the air flow direction, and then the ionizer 122 may be disposed.

In the dust collecting section 127 of the dust collector case 121, the ionizer 122, the dust collecting filter 123, and the ion trap 124 are sequentially arranged in the air flow direction.

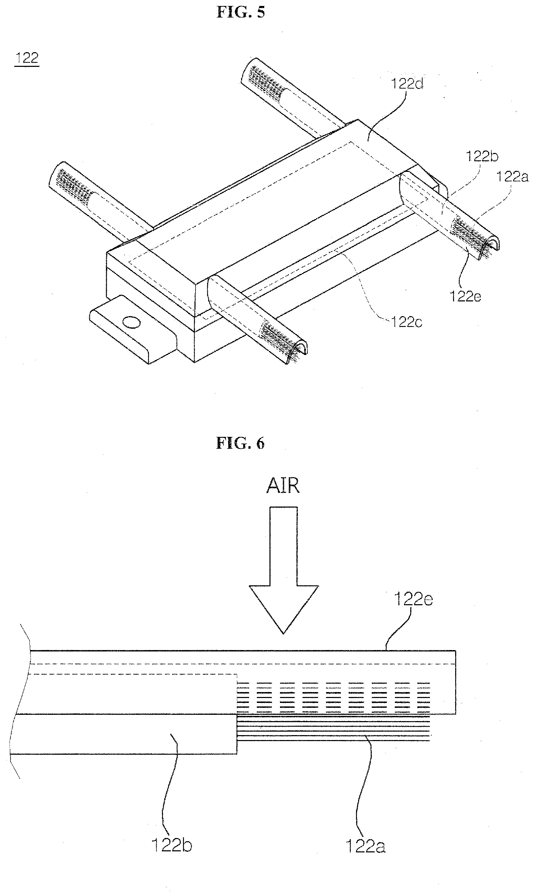

The ionizer 122 generates high voltage, causing electrical discharge at an electrode and thus ionizing molecules in the air. The ions produced by the ionizer 122 charges foreign substances. The ionizer 122 is disposed in the middle of the dust collecting section 127. A detailed description of the ionizer 122 will be given later with reference to FIGS. 5 and 6.

The charged foreign substances are collected in the dust collecting filter 123. The dust collecting filter 123 is disposed at the back of the ionizer 122 along the air flow direction. The dust collecting filter 123 is formed of a resin material having small channels through allowing air to flow therethrough.

The dust collecting filter 123 is positively and/or negatively charged. A positively charged portion of the dust collecting filter 123 captures negatively charged foreign substances, while a negatively charged portion of the dust collecting filter 123 captures positively charged foreign substances.

As the dust collecting filter 123 is charged, the dust collecting filter 123 is preferably spaced a proper distance from the ionizer 122 such that the dust collecting filter 123 does not affect electrical discharge of the ionizer 122.

The ion trap 124 collects ions generated by the ionizer 122. The ion trap 124 is formed by a grounded metallic body to collect ions. The ion trap 124 is formed in the shape of a metallic mesh. The ions are reduced in the ion trap 124. The ion trap 124 collects and removes ions which are not used in charging foreign substances to prevent the ions from adversely affecting human health.

The ion trap 124 is disposed at the back of the dust collecting filter 123 along the air flow direction. Depending on embodiments, the ion trap 124 may be disposed at the front of or in the dust collecting filter 123.

The ion trap 124 may be disposed close to and spaced apart from the dust collecting filter 123. The distance between the ionizer 122 and the dust collecting filter 123 is greater than the distance between the dust collecting filter 123 and the ion trap 124.

Depending on embodiments, the ion trap 124 may be omitted and instead the heat exchanger 140 formed of a metallic material may be used. That is, the heat exchanger 140 may be grounded such that ions are collected by the heat exchanger 140.

FIG. 5 is a perspective view showing an ionizer according to an embodiment of the present invention, and FIG. 6 is a detailed view showing a part of the ionizer shown in FIG. 5.

The ionizer 122 according to the illustrated embodiment includes a circuit 122c to generate high voltage, a circuit case 122d to surround the circuit 122c, an electrode 122a disposed outside of the circuit case 122d to discharge electricity according to high voltage generated by the circuit 122c to ionize molecules in the air, an electrode support tube 122b to connect the circuit case 122d to the electrode 122a, and an electrode cover 122e disposed at a portion around the electrode 122a.

The circuit 122c is a circuit to generate high voltage to allow the electrode 122a to discharge electricity. The circuit may generate alternating current, positive or negative direct current, or pulsed direct current at high voltage and supply the same to the electrode 122a. In the illustrated embodiment, the circuit 122c is a constant voltage circuit that generates negative direct current. The circuit 122c preferably has an output voltage of -7kVp±8%, an output frequency of 110Hz±10%, and a duty of 15% to 25%. The circuit 122c includes a circuit board and various electronic devices.

The circuit case 122d surrounds the circuit 122c to protect the circuit 122c and prevent electric leakage in the circuit 122c. The circuit case 122d is preferably formed of a plastic material and in the shape of a rectangular parallelepiped. Molding of silicone rubber may be performed inside of the circuit case 122d to protect the circuit 122c.

The electrode support tube 122b protrudes from the circuit case 122d to support the electrode 122a. The electrode support tube 122b is formed in the shape of a stick. One end of the electrode support tube 122b is connected to the circuit case 122d, and the other end is provided with the electrode 122a. The electrode support tube 122b includes a wire to transfer the high voltage generated by the circuit 122c to the electrode 122a, and a sheath formed of a plastic material to surround the outer side of the wire. The electrode support tube 122b electrically connects the circuit 122c to the electrode 122a.

A plurality of electrode support tubes 122b may be disposed depending on the number of the electrodes 122a. In the illustrated embodiment, four electrode support tubes 122b spaced from each other are disposed around the circuit case 122d.

The electrode 122a discharges electricity, thereby ionizing molecules in the air. When high voltage generated by the circuit 122c is applied to the electrode 122a via the electrode support tube 122b, the electrode 122a discharges electricity, ionizing molecules in the air. When high voltage is applied to the electrode 122a, negative ions such as OH- and O- or positive ions such as H+ are produced.

The electrode 122a may be formed of carbon fiber. If the electrode 122a is formed of superfine carbon fibers, ions are produced by corona discharge. The electrode 122a is preferably in the shape of a brush formed by binding hundreds of superfine carbon fibers having a diameter of a few micrometers to the electrode support tube 122b. In the illustrated embodiment, the electrode 122a is formed in the shape of a brush having about 1000 carbon fibers whose diameter is about 7 µm.

In the electrode 122a formed into a brush by binding carbon fibers, only one among hundreds of carbon fibers discharges electricity. Depending on embodiments, the electrode 122a may be formed in the shape of a needle, or of a mesh having a pattern. A plurality of electrodes 122a may be disposed. In the illustrated embodiment, four electrodes 122a are disposed.

Preferably, the electrodes 122a are properly spaced from each other to minimize mutual interference between the electrodes 122a. Preferably, the electrodes 122a are equally spaced from each other in an imaginary plane perpendicular to the air flow direction, and are symmetrically disposed with respect to a horizontal line and/or a vertical line.

Ions generated at the electrodes 122a charge foreign substances. Negative ions provide electrons to foreign substances, thus negatively charging the foreign substances, while positive ions remove electrons from foreign substances, thus positively charging the foreign substances.

The electrode cover 122e covers one side of the electrodes 122a facing the air flowing thereto. The electrode cover 122e is disposed at a position around the electrode 122a toward which air flows. The electrode cover 122e is disposed around the electrode 122a to face the air suction portion 116.

The electrode cover 122e is formed of a plastic material and in the shape of a semicircular tube. One side of the electrode cover 122e is connected to the circuit case 122d. The electrode cover 122e having a semicircular shape surrounds half of the circumferences of the electrode support tube 122b and the electrodes 122a. Depending on embodiments, the electrode cover 122e may be formed in various shapes. The electrode cover 122e may be formed in a cylindrical shape to surround the entire circumference of the electrode support tube 122b and leave a part of the circumference of the electrodes 122a uncovered.

The electrode cover 122e prevents any conductive material put through the air suction portion 116 by a user from contacting the electrodes 122a, and also prevents foreign substances contained in the flowing air from hitting the electrodes 122a. The portion of the electrode 122a facing in the direction in which air flows is open to the outside to generate ions.

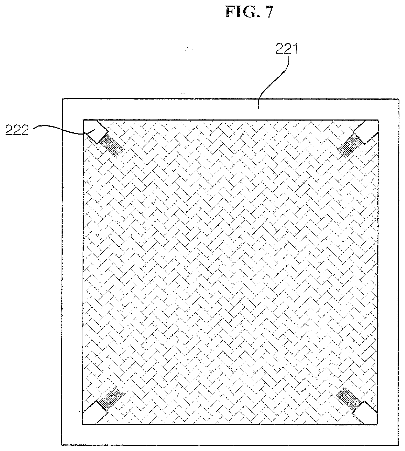

FIG. 7 is a front view showing a dust collector according to another embodiment of the present invention.

In the illustrated embodiment, four electrodes 222 are provided and disposed at four corners of a dust collector case 221 having a rectangular cross section. Four electrode support tubes (not shown) are also provided. In this case, an electrode cover (not shown) is preferably disposed around the electrodes 222.

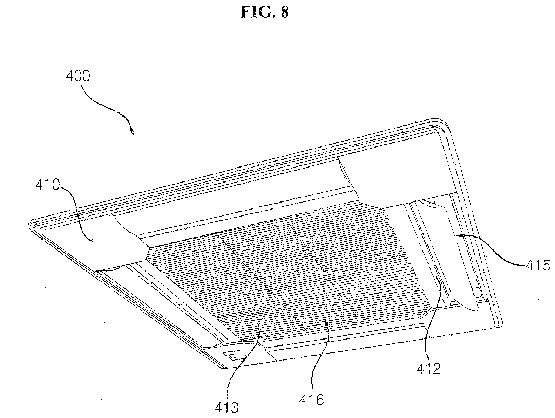

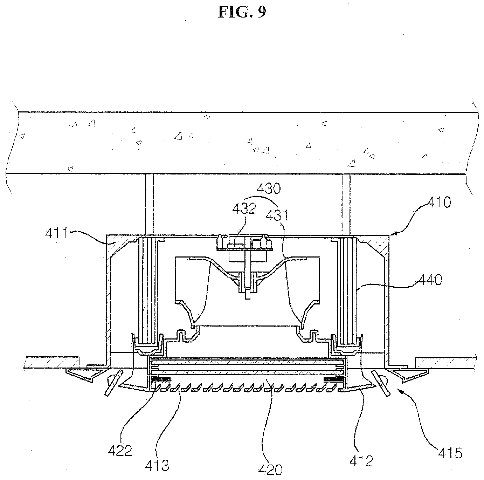

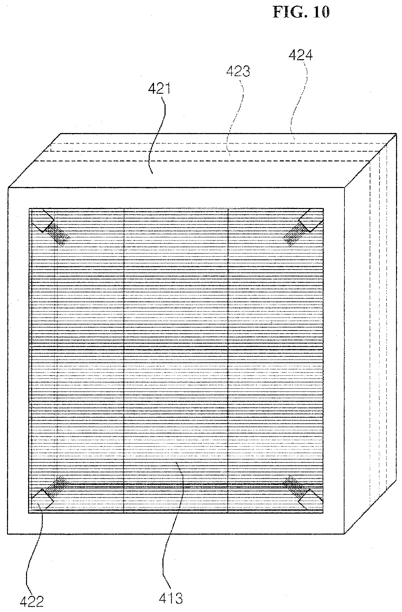

FIG. 8 is a perspective view showing an air conditioner according to another embodiment of the present invention, FIG. 9 is a cross-sectional view showing the air conditioner shown in FIG. 8, and FIG. 10 is a perspective view showing a dust collector according to anther embodiment of the present invention.

The air conditioner 400 according to the illustrated embodiment includes a cabinet 410, a dust collector 420 disposed inside the cabinet 410 to eliminate foreign substances in the flowing air, an air blower 430 to cause the air to flow, and a heat exchanger 440 to cause heat to be exchanged between the air blown by the air blower 430 and a refrigerant to adjust temperature. In the illustrated embodiment, the air conditioner 400 is a ceiling-mounted indoor unit.

The cabinet 410 includes a cabinet body 411, a suction panel 413 coupled to the central part of the lower portion of the cabinet body 411 and provided with an air suction portion 416 allowing external air to be suctioned into the cabinet 410 therethrough, and a discharge panel 412 coupled to the periphery of the lower portion of the cabinet body 411 and provided with an air discharge portion 415 allowing the air in the cabinet 410 to be discharged to the outside therethrough.

The air blower 430 includes a motor 432 to generate rotational power, and a fan 431 rotated by the motor 432.

The heat exchanger 440 causes heat to be exchanged between air and a refrigerant to cool or heat the air and is disposed around the air blower 430.

The dust collector 420 includes a dust collector case 421 forming a flow channel allowing air to flow therethrough, an electrode 422 formed of carbon fiber to ionize molecules in the air, a charged dust collecting filter 423 to collect foreign substances charged by ions produced by the electrode 422, a grounded ion trap 424 to collect ions produced by the electrode 422. Preferably, an electrode cover (not shown) is disposed around the electrode 422. The electrode cover (not shown) is disposed at the side of the suction panel 413 around the electrode 422.

Preferably, the cross section of the dust collector case 421 is formed to correspond to the suction panel 413 such that the dust collector case 421 is coupled to the suction panel 413. By removing the dust collector case 421 coupled to the suction panel 413 from the cabinet body 411, the dust collecting filter 423 or the entire dust collector 420 coupled to the suction panel 413 may be replaced.

An ionizer according to the present invention has one or more of the following effects.

First, the amount of ions generated may be maximized as an electrode is formed of carbon fibers arranged in a brush shape and causing corona discharge.

Second, as an electrode cover covers the electrode, electric shock to a user may be prevented, and contact between foreign substances and the electrode may be prevented.

Third, as the ionizer is provided as a module, it can be easily applied to various air conditioners.

Fourth, the electrode to generate ions occupies a minimum space and therefore the influence thereof on air flow may be minimized.

The present invention is not limited to the above effects. Other effects not mentioned above may be clearly understood by those skilled in the art based on the claims.

## Claims

1. An ionizer (122) for use in an air conditioner comprising:
a circuit (122c) to generate high voltage;
a circuit case (122d) to surround the circuit;
an electrode (122a) disposed perpendicular to the flow direction of air outside of the circuit case and caused to discharge electricity by the high voltage generated by the circuit to ionize molecules in air; and
an electrode cover (122e) disposed at a portion around the electrode;
an electrode support tube (122b) protruding from the circuit case to support the electrode;
wherein the electrode cover (122e) is disposed around the electrode support tube,
and **characterized in that**:
the electrode cover (122e) has a shape of a semicircular tube that surrounds half of a circumference of the electrode support tube (122b) and the electrode (122a), and
the electrode cover (122e) is disposed at a side of the electrode toward which air flows such that a portion of the electrode facing in the direction in which air flows is open to an outside to generate ions.

2. The ionizer (122) according to claim 1, wherein the electrode (122a) is formed of superfine carbon fibers bound in a brush shape.

3. The ionizer (122) according to claim 1 or 2, wherein the electrode support tube (122b) is formed in a shape of a stick, connected to the circuit case (122d), at one end thereof, and provided with the electrode (122a) at the other end thereof.

4. The ionizer (122) according to claim 1 or 2, wherein, the electrode support tube (122b) transfers the high voltage generated by the circuit (122c) to the electrode (122a).

## Patentansprüche

1. Ionisator (122) zur Verwendung in einer Klimaanlage mit:
einer Schaltung (122c) zur Erzeugung einer Hochspannung;
einem Schaltungsgehäuse (122d), das die Schaltung umgibt;
einer Elektrode (122a), die senkrecht zur Strömungsrichtung der Luft außerhalb des Schaltungsgehäuses angeordnet ist und veranlasst wird, Elektrizität durch die Hochspannung zu entladen, die durch die Schaltung erzeugt wird, um Moleküle in der Luft zu ionisieren; und
einer Elektrodenabdeckung (122e), die an einem Abschnitt um die Elektrode angeordnet ist;
einer Elektrodenhalteröhre (122b), die zum Halten der Elektrode aus dem Schaltungsgehäuse vorsteht;
wobei die Elektrodenabdeckung (122e) um die Elektrodenhalteröhre angeordnet ist, und
**dadurch gekennzeichnet, dass**:
die Elektrodenabdeckung (122e) eine Form einer halbkreisförmigen Röhre aufweist, die die Hälfte eines Umfangs der Elektrodenhalteröhre (122b) und der Elektrode (122a) umgibt, und
die Elektrodenabdeckung (122e) auf einer Seite der Elektrode angeordnet ist, zu der die Luft strömt, so dass ein Abschnitt der Elektrode, der in die Richtung weist, in die die Luft strömt, nach außen offen ist, um Ionen zu erzeugen.

2. Ionisator (122) nach Anspruch 1, wobei die Elektrode (122a) aus ultrafeinen Kohlenstofffasern ausgebildet ist, die in einer Bürstenform gebunden sind.

3. Ionisator (122) nach Anspruch 1 oder 2, wobei die Elektrodenhalteröhre (122b) in einer Form eines Stabs ausgebildet ist, der an dessen einem Ende mit dem Schaltungsgehäuse (122d) verbunden ist und an dessen anderem Ende mit der Elektrode (122a) versehen ist.

4. Ionisator (122) nach Anspruch 1 oder 2, wobei die Elektrodenhalteröhre (122b) die durch die Schaltung (122c) erzeugte Hochspannung zur Elektrode (122a) überträgt.

## Revendications

1. Ioniseur (122) destiné à être utilisé dans un climatiseur, comprenant :
un circuit (122c) pour la génération d'une haute tension ;
un boîtier (122d) de circuit pour entourer le circuit ;
une électrode (122a) disposée perpendiculairement à la direction d'écoulement de l'air en dehors du boîtier de circuit et amenée à décharger de l'électricité sous l'effet de la haute tension générée par le circuit pour ioniser des molécules dans l'air ; et
une protection (122e) d'électrode disposée sur une partie autour de l'électrode ;
un tube de support (122b) d'électrode faisant saillie du boîtier de circuit pour supporter l'électrode ;
où la protection (122e) d'électrode est disposée autour du tube de support d'électrode,
**caractérisé en ce que** :
la protection (122e) d'électrode a la forme d'un tube semi-circulaire entourant la moitié de la circonférence du tube de support (122b) d'électrode et l'électrode (122a), et
la protection (122e) d'électrode est disposée sur un côté de l'électrode vers lequel s'écoule l'air, de telle manière qu'une partie de l'électrode opposée à la direction d'écoulement de l'air est ouverte vers l'extérieur pour générer des ions.

2. Ioniseur (122) selon la revendication 1, où l'électrode (122a) est constituée de fibres de carbone superfines liées en brosse.

3. Ioniseur (122) selon la revendication 1 ou la revendication 2, où le tube de support (122b) d'électrode a la forme d'un bâtonnet raccordé au boîtier (122d) de circuit par une de ses extrémités, et pourvu de l'électrode (122a) à son autre extrémité.

4. Ioniseur (122) selon la revendication 1 ou la revendication 2, où le tube de support (122b) d'électrode transfère la haute tension générée par le circuit (122c) à l'électrode (122a).
